# EUROPEAN PATENT APPLICATION

(11) **EP 1 840 228 A2**
(43) Date of publication of application: **03.10.2007**
(21) Application number: 07447022.0
(22) Date of filing: 20.03.2007
(51) Int. Cl.: C12Q 1/68

(54) **FOXA1 marker for judging lymph node metastasis of breast cancer, a primer and a method for judging lymph node metastasis of breast cancer using the marker**

(30) Priority: 31.03.2006 JP 2006098713
(71) Applicant: SYSMEX CORPORATION, Kobe-shi, Hyogo 651-0073 (JP)
(72) Inventor: Kaduki, Nakabayashi, Kobe-shi, Hyogo 651-0073 (JP); Yasuhiro, Otomo, Kobe-shi, Hyogo 651-0073 (JP); Motonari, Daitho, Kobe-shi, Hyogo 651-0073 (JP); Takayuki, Takahata, Kobe-shi, Hyogo 651-0073 (JP); Nariaki, Matsuura, Kobe-shi, Hyogo 651-0073 (JP)
(74) Representative: De Clercq, Ann G. Y.

(57) **Abstract**

The object of the present invention is to provide a novel marker for a correct diagnosis of lymph node metastasis of breast cancer. This object is attained by a marker for judging lymph node metastasis of tumor cells derived from breast cancer, the marker including mRNA of the gene encoding Forkhead box A1 (FOXA1) or a fragment thereof.

SEQUENCE LISTING

<110>  Sysmex Corporation

<120>  A marker for judging lymph node metastasis of breast cancer,
a primer and a method for judging lymph node metastasis of breast
cancer using the marker

<130>  SYSM-010-EP

<150>  JP 2006-098713
<151>  2006-03-31

<160>  5

<170>  PatentIn version 3.1

<210>  1
<211>  20
<212>  DNA
<213>  Artificial

<220>
<223>  Primer

<400>  1
atggttgtat tgggcagggt                                                20

<210>  2
<211>  20
<212>  DNA
<213>  Artificial

<220>
<223>  Primer

<400>  2
accagtccca ctaagcagcc                                                20

<210>  3
<211>  3124
<212>  DNA
<213>  Homo sapiens

<400>  3
taagatccac atcagctcaa ctgcacttgc ctcgcagagg cagcccgctc acttcccgcg      60

gaggcgctcc ccggcgccgc gctccgcggc agccgcctgc ccccggcgct gcccccgccc     120

gccgcgccgc cgccgccgcc gcgcacgccg cgccccgcag ctctgggctt cctcttcgcc     180

cgggtggcgt tgggcccgcg cgggcgctcg ggtgactgca gctgctcagc tcccctcccc     240

cgccccgcgc cgcgcggccg cccgtcgctt cgcacagggc tggatggttg tattgggcag     300

ggtggctcca ggatgttagg aactgtgaag atggaagggc atgaaaccag cgactggaac     360

agctactacg cagacacgca ggaggcctac tcctccgtcc cggtcagcaa catgaactca     420

ggcctgggct ccatgaactc catgaacacc tacatgacca tgaacaccat gactacgagc     480

ggcaacatga ccccggcgtc cttcaacatg tcctatgcca acccgggcct aggggccggc     540

ctgagtcccg gcgcagtagc cggcatgccg gggggctcgg cgggcgccat gaacagcatg     600

actgcggccg gcgtgacggc catgggtacg gcgctgagcc cgagcggcat gggcgccatg     660

ggtgcgcagc aggcggcctc catgaatggc ctgggcccct acgcggccgc catgaacccg     720

tgcatgagcc ccatggcgta cgcgccgtcc aacctgggcc gcagccgcgc gggcggcggc     780

ggcgacgcca agacgttcaa gcgcagctac ccgcacgcca agccgcccta ctcgtacatc     840

tcgctcatca ccatggccat ccagcaggcg cccagcaaga tgctcacgct gagcgagatc     900

taccagtgga tcatggacct cttcccctat taccggcaga accagcagcg ctggcagaac 960
tccatccgcc actcgctgtc cttcaatgac tgcttcgtca aggtggcacg ctccccggac 1020
aagccgggca agggctccta ctggacgctg cacccggact ccggcaacat gttcgagaac 1080
ggctgctact tgcgccgcca gaagcgcttc aagtgcgaga agcagccggg ggccggcggc 1140
gggggcggga gcggaagcgg gggcagcggc gccaagggcg gccctgagag ccgcaaggac 1200
ccctctggcg cctctaaccc cagcgccgac tcgcccctcc atcggggtgt gcacgggaag 1260
accggccagc tagagggcgc gccggccccc gggcccgccg ccagccccca gactctggac 1320
cacagtgggg cgacggcgac agggggcgcc tcggagttga agactccagc ctcctcaact 1380
gcgcccccca taagctccgg gcccggggcg ctggcctctg tgcccgcctc tcacccggca 1440
cacggcttgg caccccacga gtcccagctg cacctgaaag gggaccccca ctactccttc 1500
aaccacccgt tctccatcaa caacctcatg tcctcctcgg agcagcagca taagctggac 1560
ttcaaggcat acgaacaggc actgcaatac tcgccttacg gctctacgtt gcccgccagc 1620
ctgcctctag gcagcgcctc ggtgaccacc aggagcccca tcgagccctc agccctggag 1680
ccggcgtact accaaggtgt gtattccaga cccgtcctaa acacttccta gctcccggga 1740
ctggggggtt tgtctggcat agccatgctg gtagcaagag agaaaaaatc aacagcaaac 1800
aaaaccacac aaaccaaacc gtcaacagca taataaaatc ccaacaacta tttttatttc 1860
atttttcatg cacaaccttt cccccagtgc aaaagactgt tactttatta ttgtattcaa 1920
aattcattgt gtatattact acaaagacaa ccccaaacca atttttttcc tgcgaagttt 1980
aatgatccac aagtgtatat atgaaattct cctccttcct tgcccccctc tctttcttcc 2040
ctctttcccc tccagacatt ctagtttgtg gagggttatt taaaaaaaca aaaaaggaag 2100
atggtcaagt ttgtaaaata tttgtttgtg ctttttcccc ctccttacct gaccccctac 2160
gagtttacag gtctgtggca atactcttaa ccataagaat tgaaatggtg aagaaacaag 2220
tatacactag aggctcttaa aagtattgaa agacaatact gctgttatat agcaagacat 2280
aaacagatta taaacatcag agccatttgc ttctcagttt acatttctga tacatgcaga 2340
tagcagatgt ctttaaatga aatacatgta tattgtgtat ggacttaatt atgcacatgc 2400
tcagatgtgt agacatcctc cgtatattta cataacatat agaggtaata gataggtgat 2460
atacatgata cattctcaag agttgcttga ccgaaagtta caaggacccc aacccctttg 2520
tcctctctac ccacagatgg ccctgggaat caattcctca ggaattgccc tcaagaactc 2580
tgcttcttgc tttgcagagt gccatggtca tgtcattctg aggtcacata acacataaaa 2640
ttagtttcta tgagtgtata ccatttaaag aatttttttt tcagtaaaag ggaatattac 2700
aatgttggag gagagataag ttatagggag ctggatttca aaacgtggtc caagattcaa 2760
aaatcctatt gatagtggcc attttaatca ttgccatcgt gtgcttgttt catccagtgt 2820
tatgcacttt ccacagttgg acatggtgtt agtatagcca gacgggtttc attattattt 2880
ctctttgctt tctcaatgtt aatttattgc atggtttatt ctttttcttt acagctgaaa 2940
ttgctttaaa tgatggttaa aattacaaat taaattgtta atttttatca atgtgattgt 3000
aattaaaaat attttgattt aaataacaaa aataatacca gattttaagc cgtggaaaat 3060
gttcttgatc atttgcagtt aaggacttta aataaatcaa atgttaacaa aaaaaaaaaa 3120
aaaa 3124

<210>  4
<211>  20
<212>  DNA
<213>  Artificial

<220>
<223>  Primer

<400>  4
ccacactgtg cccatctacg                                                    20


<210>  5
<211>  26
<212>  DNA
<213>  Artificial

<220>
<223>  Primer

<400>  5
aggatcttca tgaggtagtc agtcag                                             26

## Description

### FIELD OF THE INVENTION

The present invention relates to a marker for judging lymph node metastasis of breast cancer, a primer for amplifying a cDNA derived from the marker, and a method for judging lymph node metastasis of breast cancer using the marker.

### BACKGROUND

Detection of tumor cells in a lymph node for diagnosis of breast cancer (diagnosis of lymph node metastasis) provides useful informations for determining surgical sites and for decision to institute postoperative chemotherapy. Currently, most pathologists make their diagnosis of lymph node metastasis by biopsy such as, for example, HE (hematoxylin-eosin) stain or IHC (immunohistochemical) method using a frozen or paraffin section of lymph node tissue resected from a living body. However, even if tumor cells are present in the lymph node, pathologists may fail to recognize the tumor cells when preparing a section containing no tumor cells and performing a biopsy with the section.

Recently, in view of the current situation, an increasing number of molecular diagnostic studies for cancer have been conducted using LAMP (loop-mediated isothermal amplification) method, PCR (polymerase chain reaction) method and the like. A molecular diagnosis can be performed by measuring a molecular marker such as, for example, a protein specifically expressed in tumor cells, a gene encoding the protein, or mRNA of the gene. It is known that cytokeratin 19 (CK19) and human carcinoembryonic antigen (CEA) are useful as a molecular marker for judging lymph node metastasis of breast cancer (hereinafter, also simply referred to as a marker). The expression amounts of these proteins in normal lymph node are found to be significantly different from those in breast cancer cells metastasized to lymph node.

Various proteins other than the above markers are also expressed in breast cancer cells. Accordingly, the screening of a molecule that can be used as a marker has increasingly been undertaken. It has been reported that the proteins such as Forkhead box A1 (FOXA1) are highly expressed in breast cancer cells (EA Williamson et al., BRCA1 and FOXA1 proteins coregulate the expression of the cell cycle-dependent kinase inhibitor p27Kip1; Oncogene (2005), p.1-9).

### SUMMARY

**The scope of the present invention is defined solely by the appended claims, and is not affected to any degree by the statements within this summary.**

The expression amounts of the above proteins reported by EA Williamson et al. are unknown in normal lymph node cells. Therefore, it is also unknown which proteins among the above proteins are useful as a marker for lymph node metastasis. Moreover, there is no description of using the mRNA for the protein that is highly expressed in breast cancer cells as a marker for lymph node metastasis.

The present invention has been made in view of the above circumstances. An object of the present invention is to provide a novel marker for the diagnosis of lymph node metastasis of breast cancer. Another object of the present invention is to provide a primer for amplifying cDNA derived from the marker. Yet another object of the present invention is to provide a method for judging lymph node metastasis of breast cancer using the maker.

The present invention relates to a marker for judging lymph node metastasis of tumor cells derived from breast cancer, and the marker includes mRNA of the gene encoding Forkhead box A1 (FOXA1) or a fragment thereof.

The present invention also relates to a primer for nucleic acid amplification for measuring the above marker, and the primer includes: (a) a polynucleotide having a sequence as set forth in SEQ ID NO:1 or SEQ ID NO:2; and (b) a polynucleotide having a sequence with substitution, deletion, insertion or addition of at least one nucleotide with respect to the polynucleotide in (a) and functioning as a primer in a nucleic acid amplification reaction. The present invention also relates to a primer set for measuring the marker.

Further, the present invention relates to a method for judging lymph node metastasis of breast cancer including the steps of: measuring a marker including mRNA of the gene encoding FOXA1 or a fragment thereof in a sample prepared from a lymph node; and judging metastasis of tumor cells derived from breast cancer to the lymph node, based on the results of the measurement. More specifically, lymph node metastasis has occurred when the marker is excessively present in the sample.

According to the present invention, a novel marker that may be used for diagnosis of lymph node metastasis of breast cancer can be provided. Furthermore, according to the present invention, a primer for amplifying cDNA derived from the marker can be provided. Moreover, according to the present invention, a method for judging lymph node metastasis of breast cancer using the maker can be provided.

### BRIEF DESCRIPTION OF THE DRAWINGS

Fig. 1 is a graph showing the results of measuring PCR threshold cycle numbers at which a fluorescence intensity obtained by RT-PCR using each negative and positive specimen has reached a certain intensity (PCR threshold cycle number for negative and positive specimens, respectively) in Example 1; and
Fig. 2 is graphs showing PCR threshold cycle number (Ct) at which each fluorescence intensity obtained by RT-PCR amplification of FOXA1 or beta-actin mRNA in the samples from the lymph nodes of patients with and without confirmed metastasis to the lymph node has reached a certain intensity.

### DETAILED DESCRIPTION OF THE EMBODIMENT

The marker of the present invention is mRNA of the gene encoding the protein called FOXA1 which is present in excess in tumor cells derived from breast cancer, or a part of the mRNA. By measuring this marker, the presence of tumor cells in lymph node is measured. As used herein, "being present in excess" refers to being present at a higher level than in the normal lymph node cells. The term "measuring" includes not only measuring the presence or absence of an analyte, but also quantifying. The term "mRNA" includes not only a mature mRNA, but also a pre-mRNA (such as mRNA transcripts prior to splicing or polyadenylation).

Since various kinds of proteins are expressed in a breast cancer cell, it is unable to determine whether an mRNA encoding a specific protein is useful as a marker for lymph node metastasis by merely proving that the protein is contained in a large amount in the tumor cell. A useful marker for lymph node metastasis is an mRNA or a part thereof encoding the protein, among various proteins being expressed in a tumor cell, which has been found to be present in excess at a higher level in lymph node cells that have metastasized from breast cancer than in normal lymph node cells.

When trying to measure a marker, it is preferred to prepare a measurement sample. As a measurement sample, solubilized lymph node cells obtained from a living body can be used. The samples containing lymph node cells include, for example, a cell mass containing lymph node cells removed by surgery, a sample containing lymph node cells obtained by biopsy, and the like.

The measurement sample can be prepared, for example, as follows. First, a reagent for solubilization (hereinafter, referred to as "a solubilization solution") is added to the lymph node cells. The cells in the solubilization solution are broken by homogenization or the like so as to isolate the molecules inside the cell membrane into the solution. The resulting homogenate is then centrifuged and the supernatant is collected for use as a measurement sample. It is to be noted that a process such as nucleic acid purification or nucleic acid extraction may be performed before and/or after the centrifugation.

In order to measure the marker of the present invention which may be contained in the resulting measurement sample, it is preferable to conduct the following steps: adding a primer capable of measuring the marker, an enzyme having a reverse transcriptional activity, and a DNA polymerase to the measurement sample so as to prepare a reaction solution; performing nucleic acid amplification; and measuring the amplified cDNA in the reaction solution. The nucleic acid amplification method is not particularly limited, and known methods such as PCR method and LAMP method can be used. Since the marker is an RNA, the nucleic acid amplification method in which a reverse transcription reaction is followed by the nucleic acid amplification reaction (for example, RT-PCR method and RT-LAMP method) can be used. In such nucleic acid amplification method, a cDNA is reverse transcribed from the marker mRNA as a template, and by using the obtained cDNA as a template, nucleic acid amplification reaction can be proceeded.

The conditions for the reverse transcription reaction and the nucleic acid amplification reaction can be appropriately modified depending on the cDNA and primer sequences corresponding to the marker of the present invention that is a template. The conditions that can be used for the reverse transcription reaction and the nucleic acid amplification reaction is set forth, for example, in Sambrook, J. et al. (1989) Molecular Cloning: A Laboratory Manual (2nd ed.), Cold Spring Harbor Laboratory Press, New York.

The sequence of the primer for measuring the marker is not particularly limited as long as the primer is a polynucleotide that can be used to amplify the cDNA corresponding to the marker. The length of the primer is preferably 5 to 100 nucleotides, and more preferably 10 to 50 nucleotides. The primer can be synthesized by a method of nucleic acid synthesis known in the art.

As long as having a primer function, the above primer may contain mutation at least one nucleotide such as substitution, deletion, insertion and addition. By "primer function" is meant a function to hybridize to a cDNA corresponding to a marker (that is, a cDNA reverse transcribed from a marker or a complementary strand of the cDNA) and prime an extension reaction which is a part of nucleic acid amplification. The mutated polynucleotide preferably has at least 60% complementarity and more preferably at least 80% complementarity to the region of the cDNA sequence transcribed from the marker, to which the polynucleotide hybridizes. Preferably at least 3 bases from the 3' end of the polynucleotide, and more preferably at least 5 bases from the 3' end of the polynucleotide are completely complementary to the region in order to allow the polynucleotide to function as a primer.

The primer is preferably composed of:
(a) a polynucleotide having a sequence as set forth in SEQ ID NO:1 or SEQ ID NO:2; or
(b) a polynucleotide having a sequence with substitution, deletion, insertion or addition of at least one nucleotide with respect to the polynucleotide in (a) and functioning as a primer in a nucleic acid amplification reaction.

The above primer may also be used as a primer set composed of a combination of a first primer and a second primer (a forward primer and a reverse primer) that can be used to amplify the cDNA corresponding to the marker of the present invention by the nucleic acid amplification method. In this case, the primer set is desirable to include:
the first primer including
   (a) a polynucleotide having a sequence as set forth in SEQ ID NO:1; or
   (b) a polynucleotide having a sequence with substitution, deletion, insertion or addition of at least one nucleotide with respect to the polynucleotide in (a) and functioning as a primer in a nucleic acid amplification reaction; and
the second primer including
   (c) a polynucleotide having a sequence as set forth in SEQ ID NO:2; or
   (d) a polynucleotide having a sequence with substitution, deletion, insertion or addition of at least one nucleotide with respect to the polynucleotide in (c) and functioning as a primer in a nucleic acid amplification reaction.

The above primers may be modified by techniques commonly used in the art. The above primers can be labeled with radioactive elements or with nonradioactive molecules. Examples of the radioactive isotopes to be used include ³²P, ³³P, ³⁵S, ³H or ¹²⁵I. The nonradioactive materials are selected from ligands such as biotin, avidin, streptavidin or digoxigenin, haptens, dyes and luminescent agents, such as radioluminescent, chemiluminescent, bioluminescent, fluorescent or phosphorescent agents.

The enzymes having reverse transcriptase activity and DNA polymerases well known in the art can be used. Examples of the enzymes having reverse transcriptase activity include AMV (Avian Myeloblastosis Virus) reverse transcriptase and M-MLV (Molony Murine Leukemia Virus) reverse transcriptase. Examples of DNA polymerases which can be used include Taq DNA polymerase, Pfu DNA polymerase, T4 DNA polymerase and Bst DNA polymerase.

The marker can be measured by measuring the product generated through the above nucleic acid amplification. For example, the marker can be measured by measuring the amplified cDNA. The measurement of the amplified cDNA can be performed by mixing a reaction solution with a fluorescent intercalator such as ethidium bromide and SYBR Green to achieve fluorescent staining of the cDNA in the reaction solution, and measuring the fluorescence intensity of the reaction solution. The quantitative determination of the marker can also be performed by previously adding the above fluorescent intercalator to the reaction solution, and real-time measuring the fluorescence intensity of the reaction solution.

When magnesium pyrophosphate is generated as a byproduct in conjunction with the amplification of cDNA, the cDNA may also be measured by measuring the magnesium pyrophosphate. Because of the insolubility of magnesium pyrophosphate, the reaction solution becomes clouded gradually as the amount of magnesium pyrophosphate generated in the solution increases. Therefore, the cDNA can also be measured by optical measurements (for example, turbidimetric measurement and absorbance measurement) of the reaction solution. The marker can also be quantified by performing the optical measurements in real-time.

Next, a lymph node metastasis of breast cancer can be judged based on the measurement result of the above marker.

The presence of the marker of the present invention may be measured not only in tumor cells but slightly in normal cells. In such cases, the lymph node metastasis of tumor cells derived from breast cancer is preferably judged by comparing the measurement result of the above marker to a predetermined threshold.

When the marker is measured using, for example, RT-PCR in real time, the lymph node metastasis of tumor cells derived from breast cancer can also be judged by measuring the PCR threshold cycle number until reaching to a predetermined fluorescence intensity or turbidity, and comparing this measurement value with the corresponding threshold. Lymph node metastasis can also be judged by measuring fluorescence intensity or turbidity at a predetermined PCR threshold cycle number, and comparing this measurement value with a corresponding threshold.

Furthermore, when the marker is measured using, for example, RT-LAMP in real time, lymph node metastasis can also be measured by measuring the time until reaching to a predetermined fluorescence intensity or turbidity, and comparing this measurement value with the corresponding threshold. Lymph node metastasis can also be judged by measuring fluorescence intensity or turbidity at the time point when a predetermined time period has elapsed, and comparing this measurement value with a corresponding threshold. By setting a plurality of thresholds, the lymph node metastasis of tumor cells can be judged at multiple stages, for example, "most positive", "positive", "negative", and the like.

The threshold can be set to a value not larger than the value corresponding to the amount of the marker contained in a living body sample confirmed to contain tumor cells (a positive sample) and larger than the value corresponding to the amount of the marker contained in a living body sample confirmed to contain no tumor cells (a negative sample). It is preferred to set the threshold to a value at which several positive and negative samples can be distinguished with the highest confidence, based on the measured values each corresponding to the amount of the marker in the positive and negative samples.

In addition, a microarray technology can also be used for the measurement of the above marker. Specifically, a polynucleotide probe complementary to the cDNA corresponding to the marker (hereinafter, also simply referred to as a probe) is first fixed to a solid phase. A sample that contains the cDNA obtained from the marker in a measurement sample by a reverse transcription reaction is added to this solid phase so as to allow the probe to capture the cDNA. By adding hereto a fluorescence intercalator, a hybrid of the probe and the cDNA is fluorescence stained, and then the fluorescence intensity is measured. The quantitative determination and the measurement of presence or absence of the marker can be performed based on the measurement result of the fluorescence intensity. When the probe is shorter than the cDNA, the fluorescence signal can be enhanced by adding the probe that hybridizes to the region of the cDNA that has not hybridized with the probe.

It is to be noted that the marker may be measured by fixing a probe corresponding to the marker to a solid phase, forming a hybrid of the marker and the probe, and measuring the hybrid.

This probe can be designed and produced by the same method as described for the above primer. Examples of the probe to be used include a probe with the similar sequence to the above primer.

Using either method described above, it is possible to judge whether the marker is present in the sample excessively. When the marker is determined to be present in excess in the sample, it is judged that the tumor cells derived from breast cancer have metastasized to the lymph node.

The reagents and the like required for measuring the marker of this embodiment can be provided as a reagent kit. The kit includes at least the above primer, an enzyme having a reverse transcriptional activity, a DNA polymerase, and dNTPs. Furthermore, it is preferable that the kit includes a buffer which provides a suitable condition for the enzyme reaction.

As used herein, "measuring a marker" is meant to include not only measuring the entire region of the mRNA as the marker, but also measuring a part of the region. In this embodiment, it is preferred to amplify cDNA corresponding to a part of the region of the marker and measure the amplified product. In this case, the length of the cDNA region to be measured is preferably longer by 1 to 500 nucleotides, and more preferably by 50 to 500 nucleotides than the length of the primer. Furthermore, in the case using the above primer set, the length of the cDNA region to be amplified is preferably longer by 1 to 500 nucleotides, and more preferably by 50 to 500 nucleotides than the total length of the first and the second primers.

### EXAMPLE

### Example 1

The present inventors have searched for markers that can be used to judge a lymph node metastasis of breast cancer. First, mammary gland-associated gene expression libraries were selected from the human gene expression libraries registered in public databases. From the databases, 58 genes showing low expression in lymph node and high expression in mammary gland were selected in descending order of expression level in mammary gland. Subsequently, 58 sets of primers for measuring the mRNAs of the genes encoding the 58 proteins (hereinafter, referred to these 58 mRNAs as marker candidates) were designed.

Positive and negative samples were prepared as described below using eight lymph nodes: four of which were histologically confirmed to have metastasis of tumor cells from breast cancer (positive lymph nodes); and another four of which were confirmed not to have (negative lymph nodes).

First, 4 mL of solubilization solution containing 200 mM glycine-HCl, 5% Brij35 (polyoxyethylene (35) laurylether), 20% DMSO, and 0.05% KS-538 (Shin-Etsu Chemical Co.,Ltd.) was added to the each lymph node (about 100 to 300 mg) and homogenized with a blender. The each resulting homogenate was centrifuged at 10,000 x g for 1 minute at room temperature. From the each resulting supernatant (400 µL), RNA was extracted and purified using RNeasy Mini kit (manufactured by QIAGEN GmbH., catalog number 74014) to yield RNA solution. The absorbance (λ=280 nm) of the each RNA solution was measured to determine the concentration of RNA and subsequently the each solution was diluted to a concentration of 10 ng/µL. Positive and negative specimens were prepared by mixing the RNA solutions prepared from the four positive lymph nodes and the four negative lymph nodes, respectively.

Using the positive and negative specimens obtained as described above together with the above 58 sets of primers, real-time RT-PCR was performed using ABI real-time PCR Instruments (Prism 7000) to measure the 58 mRNAs.

Real-time RT-PCR was performed using Quanti-Tect SYBR Green RT-PCR Kit (manufactured by QIAGEN GmbH., catalog number 204245) as a quantitative RT-PCR kit according to the manufacturer's instructions. The composition of the reaction solution and the reaction condition are as follows.

| Reaction Solution: | |
|---|---|
| RNase free H₂O | 10.99µL |
| 2 x Mix | 12.50µL |
| 100µM Forward Primer (final concentration, 500nM) | 0.13µL |
| 100µM Reverse Primer (final concentration, 500nM) | 0.13µL |
| Quanti-Tect RT Mix | 0.25µL |
| Positive or negative specimen | 1.00µL |
| Total | 25.00µL |

### Reaction Condition

50°C, 30 minutes
95°C, 15 minutes
PCR: with the following steps repeated for 40 cycles;
   94°C, 15 seconds,
   60°C, 1 minute.

PCR threshold cycle numbers at which a fluorescence intensity for each negative and positive specimen has reached a certain intensity (PCR threshold cycle number for negative and positive specimens, respectively) were measured by RT-PCR under the above condition. When the PCR threshold cycle number for the negative specimen is larger and that for the positive specimen is smaller, the expression level of the gene in the negative specimen is lower and that in the positive specimen is higher. That is, it is meant that the expression level of the gene is specifically increased in a lymph node with metastasis.

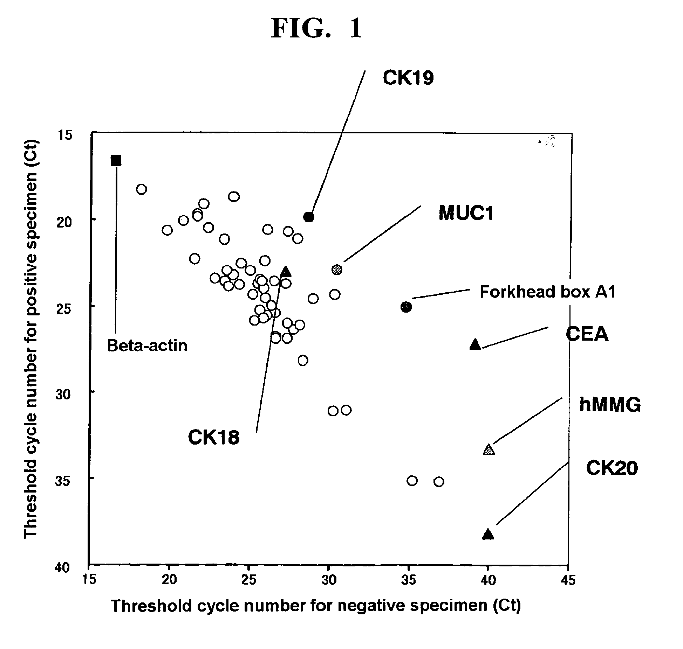

Fig. 1 shows the graph in which PCR threshold cycle numbers for positive and negative specimens are plotted on the vertical and horizontal axes, respectively. From the results shown in Fig. 1, it was found that FOXA1-encoding mRNA which has not conventionally been known as a marker for lymph node metastasis of breast cancer is useful as a marker. The sequence of FOXA1 mRNA is as shown in SEQ ID NO:3. The mRNA sequence of SEQ ID NO:3 is represented by the mRNA sequence that replaced U (uracil) with T (thymine). This sequence is available from GenBank (http://www.ncbi.nlm.nih.gov/Genbank/index.html) under accession number NM_004496.

It is to be noted that beta-actin (ACTB), used as a control, is a protein encoded by a housekeeping gene and is expressed abundantly in various cells. Therefore, the PCR threshold cycle numbers for both negative and positive specimens are small.

It is also shown that, when using CK19 mRNA which has conventionally been known as a marker for lymph node metastasis of breast cancer, the PCR threshold cycle number for positive specimen is relatively low and that for negative specimen is relatively high.

The primers used for measuring the above FOXA1 marker and the corresponding SEQ ID NOs are as follows:
Forward primer: 5'-atggttgtattgggcagggt-3' (SEQ ID NO:1);
Reverse primer: 5'-accagtcccactaagcagcc-3' (SEQ ID NO:2).

### Example 2

Fifteen RNA solutions (positive specimens) were prepared from 15 lymph nodes in which metastasis of the tumor cells derived from breast cancer was histologically confirmed. In addition, fifteen RNA solutions (negative specimens) were prepared from 15 lymph nodes in which metastasis of the tumor cells derived from breast cancer was not histologically confirmed. Using each of the 15 positive specimens and 15 negative specimens, real-time RT-PCR was performed to measure FOXA1. The method for preparing RNA solution and the RT-PCR condition are the same as in Example 1. The primers used in RT-PCR for measuring FOXA1 are the primers having SEQ ID NO:1 and SEQ ID NO:2.

RT-PCR was performed on the above positive and negative specimens using, instead of the primers for measuring FOXA1, the primers for amplifying beta-actin mRNA having the following sequences:
Forward primer: 5'-ccacactgtgcccatctacg-3' (SEQ ID NO:4);
Reverse primer: 5'-aggatcttcatgaggtagtcagtcag-3' (SEQ ID NO:5).
   It is known that beta-actin is a protein encoded by a housekeeping gene and expressed in almost all tissues. RT-PCR was also performed in the same manner as described above using the reaction solution which was not mixed with the RNA-containing specimen but pure water as a negative control (NC).

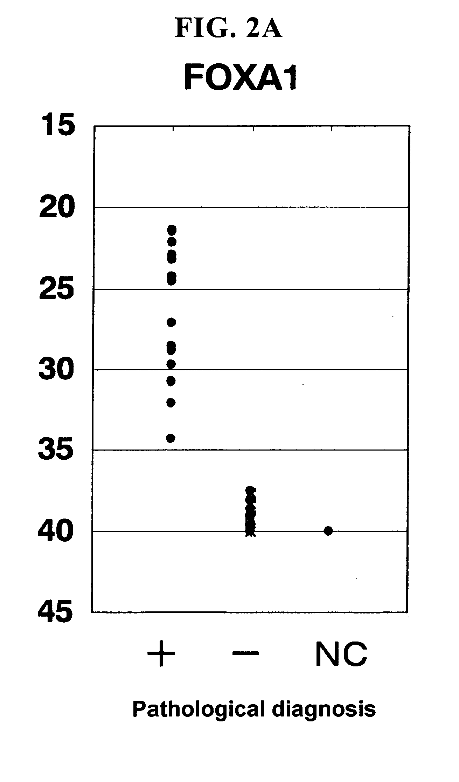

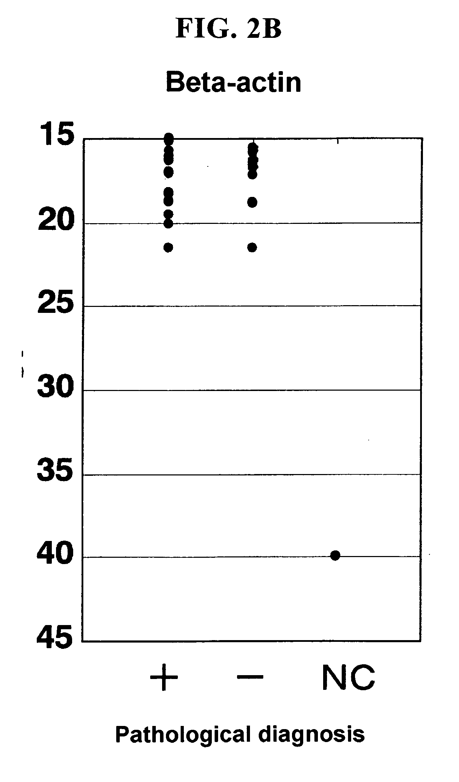

In these real-time RT-PCRs, the results of measuring the PCR threshold cycle numbers (Ct) until reaching to a certain fluorescence intensity are shown in Fig. 2A and 2B. Figure 2A is a graph showing threshold cycle numbers obtained from the measurement of FOXA1 mRNA as a marker in NC, positive and negative samples using the primers for measuring FOXA1 mRNA. Figure 2B is a graph showing threshold cycle numbers obtained from the measurement of beta-actin mRNA in NC, positive and negative samples using the primers for measuring beta-actin mRNA.

The result of Fig. 2A shows that the threshold cycle number required for amplification of a cDNA corresponding to FOXA1-encoding mRNA was low in the samples histologically confirmed to have lymph node metastasis. On the other hand, in the samples without confirmed lymph node metastasis, higher threshold cycle number was required for amplification. Furthermore, Fig. 2B shows that threshold cycle numbers required for measuring beta-actin mRNA were similar either using the negative or positive samples. Therefore, it was confirmed that the nucleic acid concentrations in the samples used were not extremely different from each other. It was also confirmed that the amplification of DNA observed with the positive sample was not due to a non-specific reaction such as primer-dimer formation.

From the above-mentioned results, it can be said that the marker for lymph node metastasis of the present invention can be preferably used for judging metastasis of breast cancer cells to lymph node.

The results of Fig. 2A and 2B also show that all the positive and negative samples used in this Example can be judged as positive and negative, respectively, by setting the threshold cycle number so as to range from 35 to 37 as the threshold for measurement of FOXA1 mRNA.

It can be judged whether the tumor cells derived from breast cancer have metastasized to the lymph node by comparing the above-mentioned threshold to the threshold cycle number measured for the sample prepared from the lymph node to which tumor cells have metastasized or not is unknown.

It is also possible to judge lymph node metastasis with higher accuracy by combining the marker of the present invention with one or more of conventional markers for lymph node metastasis of breast cancer (for example, CK19 and CEA).

The foregoing detailed description and examples have been provided by way of explanation and illustration, and are not intended to limit the scope of the appended claims. Many variations in the presently preferred embodiments will be obvious to one of ordinary skill in the art, and remain within the scope of the appended claims and their equivalents.

## Claims

1. A marker for judging lymph node metastasis of tumor cells derived from breast cancer,
the marker comprising an mRNA of a gene encoding Forkhead box A1 (FOXA1) or a fragment thereof.

2. A primer for nucleic acid amplification for measuring a marker comprising an mRNA of a gene encoding Forkhead box A1 (FOXA1) or a fragment thereof,
the primer comprising a polynucleotide selected from the group consisting of:
(a) a polynucleotide having a sequence as set forth in SEQ ID NO:1 or SEQ ID NO:2; and
(b) a polynucleotide having a sequence with substitution, deletion, insertion or addition of at least one nucleotide with respect to the polynucleotide in (a) and functioning as a primer in a nucleic acid amplification reaction.

3. The primer according to claim 2, wherein the length is 5 to 100 nucleotides.

4. The primer according to claim 2 or 3, wherein at least 3 bases from the 3' end are completely complementary to the marker as a template.

5. The primer according to any one of claim 2 to 4, wherein the nucleic acid amplification reaction is selected from RT-PCR and RT-LAMP methods.

6. A reagent kit for measuring a marker comprising an mRNA of a gene encoding Forkhead box A1 (FOXA1) or a fragment thereof, wherein the kit comprises the primer according to any one of claim 2 to 5, an enzyme having a reverse transcriptional activity, a DNA polymerase, and dNTPs.

7. The reagent kit according to claim 6, further comprising at least one selected from a solubilization solution, a buffer, a primer for amplifying a housekeeping gene and a primer for amplifying mRNA of CK19 or CEA.

8. A set of primers for a nucleic acid amplification including the primer according to any one of claim 2 to 5 comprising:
a first primer selected from the group consisting of
(a) a polynucleotide having a sequence as set forth in SEQ ID NO:1; and
(b) a polynucleotide having a sequence with substitution, deletion, insertion or addition of at least one nucleotide with respect to the polynucleotide in (a) and functioning as a primer in a nucleic acid amplification reaction; and
a second primer selected from the group consisting of
(c) a polynucleotide having a sequence as set forth in SEQ ID NO:2; and
(d) a polynucleotide having a sequence with substitution, deletion, insertion or addition of at least one nucleotide with respect to the polynucleotide in (c) and functioning as a primer in a nucleic acid amplification reaction.

9. A method for judging lymph node metastasis of breast cancer comprising the steps of:
measuring a marker including mRNA of a gene encoding Forkhead box A1 (FOXA1) or a fragment thereof in a sample prepared from a lymph node; and
judging metastasis of tumor cells derived from breast cancer to the lymph node has occurred when the measured marker is present in excess.

10. The method according to claim 9, wherein the judging step is performed so as to judge metastasis of tumor cells derived from breast cancer to the lymph node by comparing one or more predetermined thresholds and the results of the measurement.

11. The method according to claim 9 or 10, wherein the measuring step is performed so as to measure a product generated in conjunction with a nucleic acid amplification reaction.

12. The method according to any one of claim 9 to 11, wherein the measuring step comprises the steps of:
performing a reverse transcription reaction and a nucleic acid amplification reaction using a primer comprising a polynucleotide selected from the group consisting of:
(a) a polynucleotide having a sequence as set forth in SEQ ID NO:1 or SEQ ID NO:2; and
(b) a polynucleotide having a sequence with substitution, deletion, insertion or addition of at least one nucleotide with respect to the polynucleotide in (a) and functioning as a primer in a nucleic acid amplification reaction, and
measuring a product generated in conjunction with the nucleic acid amplification reaction.

13. The method according to any one of claim 9 to 12, wherein the judging step is performed so as to judge lymph node metastasis of tumor cells derived from breast cancer at multiple stages.
